# EUROPÄISCHE PATENTANMELDUNG

(11) **EP 3 566 681 A1**
(43) Veröffentlichungstag der Anmeldung: **13.11.2019**
(21) Anmeldenummer: 18171473.4
(22) Anmeldetag: 09.05.2018
(51) Int. Cl.: A61F 7/02, A61F 7/10, A61F 7/00

(54) **KÜHLPAD UND VERWENDUNG EINES KÜHLPADS ZUR KÜHLUNG VON KÖRPERTEILEN**

(71) Anmelder: Nakladal, Rastislav, 82108 Bratislava (SK)
(72) Erfinder: NAKLADAL, Ratislav, 82108 Bratislava (SK)
(74) Vertreter: Jeck, Anton

(57) **Zusammenfassung**

Die vorliegende Erfindung betrifft ein Kühlpad zur Kältebehandlung von Körperoberflächen mit einem Beutel, welcher mit mindestens einer Kammer gebildet ist. Erfindungsgemäß ist es vorgesehen, dass die mindestens eine Kammer mit einem Superabsorber für Wasser gefüllt ist und dass der Beutel von außen nach innen wasserdurchlässig ist. Weiterhin betrifft die vorliegende Erfindung die Verwendung eines Kühlpads zur Anwendung bei der Behandlung von Schwellungen, Entzündungen und kühlbedürftigen Körperpartien.

## Beschreibung

Die vorliegende Erfindung betrifft ein Kühlpad zur Kältebehandlung von Körperoberflächen.

Gattungsgemäß weist ein solches Kühlpad einen Beutel auf, welcher mit mindestens einer Kammer gebildet ist.

In der aktuellen Zeit sind Menschen und Tiere, insbesondere solche mit intensiven sportlichen Aktivitäten, einer unverhältnismäßigen körperlichen Belastung ausgesetzt. Hier geht es vor allem um Rennpferde und Hochleistungssportler, die täglich trainiert und getestet werden. Von diesen Bewegungsaktivitäten sind insbesonders die Gelenke, die im Dauerbetrieb sind, betroffen.

Dies führt zu Krankheiten, die eine Schädigung des Gewebes hervorrufen können, was zu einer Entzündung führt. Während dieses komplizierten körperlichen Prozesses werden verschiedene Chemikalien in das Gewebe aufgenommen, sei es aus dem Blut oder aus beschädigten Zellen. Die Gefäße dehnen sich aus und nehmen eine große Menge von chemischen Entzündungsmediatoren und auch andere Metaboliten auf. Das betroffene Gebiet und seine Umgebung sind geschwollen, gerötet, warm und schmerzen. Wenn die Entzündung bei ihrem Ausbruch gekühlt wird, verengen sich die Blutgefäße, so dass die Zufuhr von "kontaminiertem" Blut auf die betroffene Stelle begrenzt wird und eine Schwellung überhaupt nicht auftreten kann.

Zur Behandlung von Schwellungen sind Eis-Packs sowie kalte Umschläge bekannt, welche den Nachteil aufweisen, dass sie eine gewisse Vorbereitungszeit benötigen (Eis-Packs) oder Flüssigkeit an die Umgebung abgeben.

Die vorliegende Erfindung stellt sich die Aufgabe, ein Kühlpad bereitzustellen, welches in einfacher Weise eine Kühlwirkung bereitstellt, wobei Körper und Kleidung bei der Verwendung des Kühlpads trocken bleiben.

Die Aufgabe wird erfindungsgemäß durch ein Kühlpad mit den Merkmalen des Anspruchs 1 gelöst.

Vorteilhafte Ausführungsformen der vorliegenden Erfindung sind in den abhängigen Ansprüchen angegeben.

Nach einem Grundgedanken der vorliegenden Erfindung ist ein Kühlpad bereitgestellt, welches in seinem Inneren einen Superabsorber aufweist. Der Superabsorber ist insbesondere dadurch gekennzeichnet, dass er eine besonders gute Bindewirkung gegenüber Flüssigkeiten hat, wodurch der Verlust von Flüssigkeit an die Umgebung unterbunden oder zumindest stark eingeschränkt wird. Grundsätzlich kann jedes Material erfindungsgemäß als Superabsorber verwendet werden, welches die voranstehend beschriebenen Eigenschaften aufweist.

Besonders zweckmäßig ist es, dass das Material, aus welchem der Beutel gefertigt ist, zumindest im Bereich der Kammer, welche den Superabsorber aufweist, von außen nach innen wasserdurchlässig jedoch von innen nach außen nur dampfdurchlässig sein kann.

Nach einer Weiterbildung der Erfindung ist es vorgesehen, dass der Superabsorber vernetzte Polyacrylate, insbesondere Polyacrylamid-Copolymere, und/oder Stärke-Copolymere und/oder Carboxymethylcellulose und/oder Hydroxymethylcellulose und/oder modifizierte Cellulose aufweist, insbesondere aus diesen ausgewählt ist. Acrylamide und Celluloseverbindungen sind als besonders saugstarke Verbindungsgruppen bekannt, welche Wasser und andere Flüssigkeiten besonders stark binden und an die Umgebung nicht in Form von Flüssigkeit sondern lediglich als Wasserdampf bei Erwärmung abgeben. Hierdurch ist das Zurückhalten von Flüssigkeiten in dem Kühlpad besonders vorteilhaft ermöglicht, wodurch Kleidung und Körper des Trägers des Kühlpads trocken bleiben. Grundsätzlich kann der Superabsorber ausgebildet sein, auch andere Flüssigkeiten, wie zum Beispiel Wundwasser und andere Körpersekrete, aufzunehmen.

Nach einer bevorzugten Weiterbildung der vorliegenden Erfindung ist es vorgesehen, dass die Kammern einen Anteil eines Chlorid-Salzes, insbesondere Natriumchlorid, oder Polyvinylpyrrolidon aufweisen. Diese Verbindungen sind besonders dafür bekannt, dass sie bei Kontakt mit Wasser eine schnelle Quellung der Superabsorber bewirken können.

Besonders bevorzugt ist es nach einer Weiterbildung der Erfindung, dass der Beutel zumindest einen Anteil an Polyester, vorzugsweise einen Anteil an Polyestergewebe, aufweist, besonders bevorzugt ganz aus Polyestergewebe gebildet ist. Dieser Kunststoff beziehungsweise diese Kunstfasern oder Kunstgewebe sind besonders für ihre Widerstandsfähigkeit bekannt, wobei erfindungsgemäß auch eine besonders hohe Keimbildungsresistenz erkannt wurde. Der Beutel des Kühlpads kann mehrschichtig beziehungsweise mehrlagig gebildet sein, wobei das Polyestergewebe eine der Schichten bilden kann. Zusätzlich zu der Polyesterschicht können weitere Schichten vorgesehen sein, welche zusätzliche Eigenschaften für den Beutel bereitstellen können. Insbesondere kann der Beutel mit einer Schicht beziehungsweise Lage gebildet sein, welche Polytetrafluorethylen aufweist oder aus diesem besteht. Die einzelnen Schichten des Beutels des Kühlpads können miteinander verbunden, insbesondere verschweißt oder verklebt oder lose übereinander angeordnet sein.

Besonders zweckmäßig ist nach einer Weiterbildung der vorliegenden Erfindung, dass der Beutel zumindest einen Anteil an Polytetrafluorethylen (PTFE), vorzugsweise einen Anteil an Gewebe mit Polytetrafluorethylenfasern aufweist, besonders bevorzugt aus einem Polytetrafluorethylengewebe oder einer Polytetrafluorethylenschicht gebildet ist. PTFE kann erfindungsgemäß die Eigenschaft aufweisen, dass in zumindest eine Richtung, vorzugsweise beiden Richtungen, das Material lediglich für Wasserdampf durchlässig ist. Eine solche PTFE-Schicht oder ein PTFE Gewebe kann insbesondere auf der körperzugewandten Seite des Beutels des Kühlpads vorgesehen sein. Dadurch kann zwar keine Flüssigkeit aus dem Inneren des Beutels an die Kontakthaut des Kühlpad-Trägers gelangen, eine Durchlässigkeit für Dampf und somit eine Atmungsaktivität auch zwischen Haut und Beutel ist jedoch möglich. Hierdurch kann insbesondere einer Staunässe zwischen Haut und Kühlpad vorgebeugt werden. Besonders bevorzugt ist, dass die PTFE-Schicht beziehungsweise das PTFE Gewebe auch von außen nach innen dampfdurchlässig ist, wodurch gasförmiges Wasser zwischen Kühlpad und Haut, insbesondere Schweißdampf oder Staunässe, in den Beutel gasförmig abtransportiert werden kann.

Nach einer bevorzugten Weiterbildung der vorliegenden Erfindung ist es vorgesehen, dass der Superabsorber als gepresstes Band, als eine Gitterstruktur, in Form eines Granulats oder Gels vorliegt. Grundsätzlich kann der Beutel mit mehr als einer Kammer ausgebildet sein, in welchen jeweils ein oder mehrere Superabsorber, insbesondere auch unterschiedliche Superabsorber, vorgesehen sein können. Durch die Einteilung des Beutels in mehrere Kammern kann eine gleichmäßige Verteilung des Superabsorbers in dem Beutel bewirkt werden. Bei einem Kammersystem kann ein Nachteil darin bestehen, dass der Superabsorber, insbesondere dann, wenn er nicht in vorgeformter, vorzugsweise gepresster Form, vorliegt, in einen unteren Bereich des Beutels abrutscht und sich in diesem sammelt.

Erfindungsgemäß ist es vorgesehen, dass das Kühlpad für eine Verwendung bei der Behandlung von Schwellungen, Entzündungen und kühlbedürftigen Körperpartien einsetzbar ist.

Besonders bevorzugt ist es nach einer Weiterbildung, dass mindestens die einzelnen Fasern mindestens einer Gewebeschicht mit einer hydrophoben Oberflächenschicht gebildet sind, welche eine Plasmabeschichtung ist.

Nach einer zweckmäßigen Weiterbildung der vorliegenden Erfindung ist es vorgesehen, dass mindestens ein Superabsorber und/oder der Beutel mit einem Anteil an antibakteriellen Verbindungen, insbesondere einem Edelmetall wie Silber oder mit einem Antibiotika, gebildet sind.

Eine bevorzugte Ausführungsform wird nachstehend anhand der Zeichnungen beschrieben. Es zeigen:
- Figur 1: einen Knieschoner für Mensch oder Tier mit erfindungsgemäßem Kühlpad,
- Figur 2: einen Querschnitt durch das erfindungsgemäße Kühlpad,
- Figur 3: eine dreidimensionale Ansicht auf eine röhrenförmige Ausführungsform des erfindungsgemäßen Kühlpads in 2 Ansichten und
- Figur 4: eine Draufsicht auf die röhrenförmige Ausführungsform nach Figur 3 auf eine Ober- und Unterseite des Kühlpads.

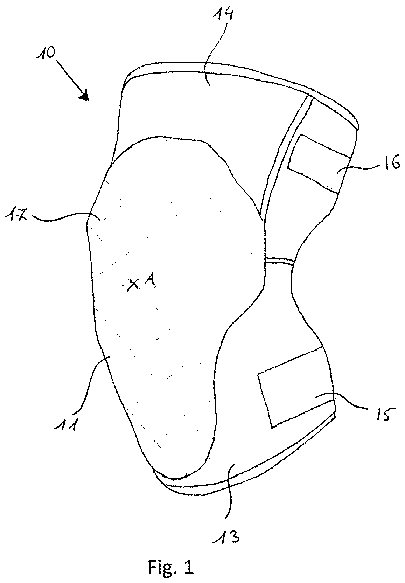

Figur 1 zeigt einen Knieschoner 10 als Halterung für das erfindungsgemäße Kühlpad 11, welches sowohl für Menschen als auch für Tiere geeignet ist und wobei sich das Kühlpad insbesondere dadurch auszeichnet, dass es an das jeweils zu kühlende Körperteil konstruktiv anpassbar ist.

Im vorliegenden Beispiel ist der Knieschoner 10 mit einem oberen Bereich 14 und einem unteren Bereich 13 gebildet, an welchen mindestens zwei Haltevorrichtungen 15 und 16 insbesondere in Form von Klettverschlüssen vorgesehen sind. Mittels der Haltevorrichtungen kann das Kühlpad an einen Umfang des zu kühlenden Körperteils anpassbar sein.

Vorliegend ist das Kühlpad 11 in einem Bereich an dem Schoner 10 angeordnet, welcher oberhalb einer Kniescheibe oder eines Ellenbogens liegt. Das Kühlpad 11 kann flächig vorgesehen sein oder als einzelne Bereiche spezifische Segmente des zu kühlenden Körperteils bedecken. Vorliegend ist das Kühlpad 11 flächig dargestellt. In etwa im Bereich des Kreuzes, welches mit dem Buchstaben A markiert ist, kann ein Ellenbogen oder ein Knie das Kühlpad von einer Innenseite des Schoners 10 kontaktieren. Vorzugsweise kann auf der dem Körper zugewandten Seite des Kühlpads 11 eine zusätzliche Textilschicht vorgesehen sein, welche einen Teil der Kühlwirkung des Kühlpads 11 abfängt, wodurch einer Unterkühlung der zu kühlenden Stelle vorgebeugt sein kann.

Grundsätzlich kann das Kühlpad 11 einstückig ausgebildet sein. Vorliegend ist das Kühlpad 11 jedoch in einzelne Kammern unterteilt, in welchen jeweils saugfähiges Material, insbesondere ein Superabsorber, vorgesehen sein kann. Die einzelnen Kammern sind vorliegend durch Nähte 17 voneinander getrennt und können beispielsweise nachträglich zur Segmentierung des Kühlpads 11 aufgebracht werden.

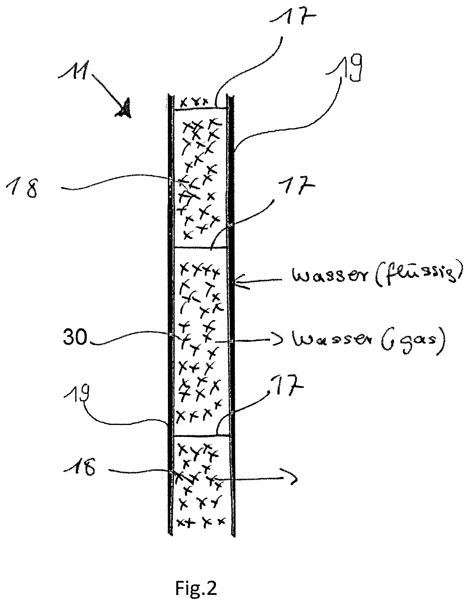

Figur 2 zeigt einen Querschnitt durch das erfindungsgemäße Kühlpad 11, welches zu beiden Seiten durch die Wand 19 des Beutels des Kühlpads 11 begrenzt ist. Als Querlinien sind durch den Querschnitt die Segmentierungen eingezeichnet, welche mittels der Nähte 17 herstellbar sind. Die Nähte können grundsätzlich mittels Nähen oder Verschweißen beziehungsweise Verkleben hergestellt sein. Zwischen den Wänden 19 ist saugfähiges Material, insbesondere ein Superabsorber, mit dem Bezugszeichen 30 angedeutet, vorgesehen. Zusammen mit dem Superabsorber können auch antibakterielle Stoffe wie beispielsweise Silber oder Antibiotika vorgesehen sein. Hierdurch kann das Kühlpad auch auf offenen Wunden verwendet werden, ohne eine Kontamination des Beutels mit pathogenem Material befürchten zu müssen.

Die Wand 19 kann ein- oder mehrlagig gebildet sein. Vorzugsweise ist die Wand aus einem Material gebildet, welches in eine Richtung für flüssiges Wasser durchlässig ist, vorzugsweise von außen nach innen, und in eine andere Richtung, vorzugsweise von innen nach außen, lediglich gasförmiges Wasser durchlässt.

Auch das Material der Wand 19 kann mit antimikrobiellen Substanzen versetzt sein, wodurch auch eine Wundsterilität förderbar sein kann.

Vor einer Verwendung kann das Kühlpad in einem Wasserreservoir gelagert werden, wodurch sich das Kühlpad innerhalb von Minuten mit Wasser füllen kann. Ist der Superabsorber in dem Kühlpad mit Wasser vollgesogen, gibt er dieses vorzugsweise lediglich durch Verdampfung wieder ab. Sollte durch entsprechenden Druck auf den Superabsorber dennoch etwas Flüssigkeit aus diesem abgesondert werden, ist die Wandung des Beutels so ausgebildet, dass sie das flüssige Wasser in dem Beutel zurückhält, wodurch eine Wiederaufnahme des ausgeschiedenen Wassers durch den Superabsorber ermöglicht sein kann.

Nach einer besonders zweckmäßigen Ausführungsform können die einzelnen Gewebefilamente mit einer Plasmabeschichtung versehen sein, welche besonders bevorzugt eine hydrophobe Oberfläche bereitstellt. Mittels einer solchen Beschichtung kann eine wasserundurchlässige jedoch dampfdurchlässige Barriere gebildet sein.

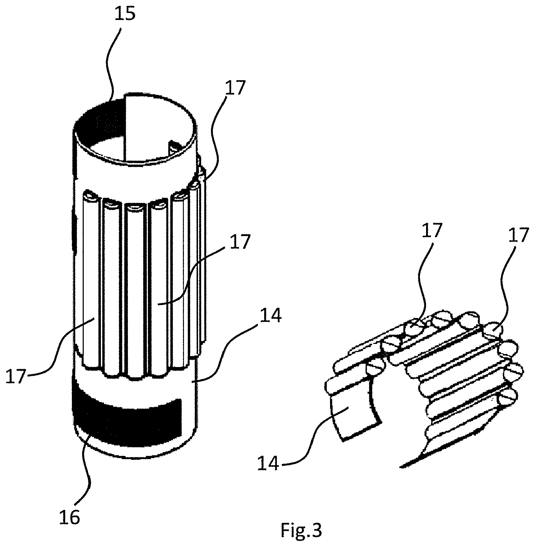

Figur 3 zeigt eine dreidimensionale Abbildung einer Ausführungsform des erfindungsgemäßen Kühlpads, bei welcher mehrere Kammern 17 bereitgestellt sind, welche röhrenförmig ausgebildet sind und in etwa parallel zueinander entlang eines Grundkörpers 14 angebracht sind. In einem oberen Bereich sind Verbindungselemente 15, vorzugsweise als Klettverschlüsse, vorgesehen, mittels welchen das im Grunde flächige Kühlpad zu einer Röhre geformt werden kann. In einem unteren Bereich ist ein weiteres Fixierungselement 16 vorgesehen. Gerade wenn das Kühlpad im Bereich von Gelenken verwendet wird, kann es notwendig sein, an gegenüberliegenden Enden des Kühlpads Vorrichtungen zum Befestigen des Kühlpads an einem Körperteil vorzusehen. Von der dreidimensionalen Draufsicht gemäß Figur 3, rechte Seite, ist ersichtlich, dass die einzelnen zylinderförmigen Kammern 17 mit einem geringfügigen Abstand zueinander gebildet sind. Zwischen den einzelnen röhrenförmigen Kammern 17 kann ein Verbindungsbereich vorgesehen sein, welcher eine erhöhte Flexibilität und Formmarke des Kühlpads ermöglicht.

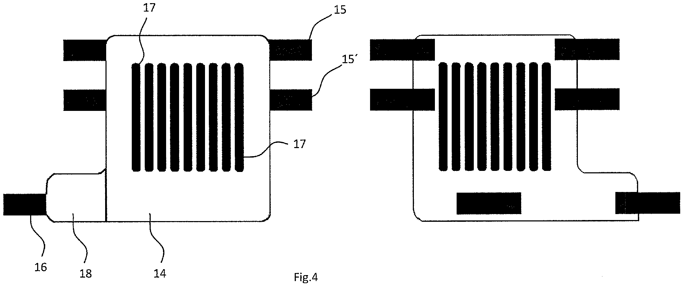

Figur 4 zeigt eine flächige Ansicht auf die Ausführungsform gemäß Figur 3 von einer Oberseite (links) und einer Unterseite (rechts). Die einzelnen Kammern 17 auf dem Grundkörper 14 können durchgängig an dem Grundkörper 14 vorgesehen sein. Demnach können die einzelnen Kammern 17 durch das Material des Grundkörpers 14 hindurchtreten, wodurch sie sowohl an der Oberseite als auch der Unterseite vorgesehen sind. Alternativ hierzu kann die Kammer wahlweise auf der Oberseite und/oder der Unterseite als jeweils separate Kammer ausgebildet sein. Vorzugsweise weist in zumindest einem Bereich der Grundkörper 14 eine Lasche 18 auf, an welcher ein weiteres Fixierungsmittel 16 vorgesehen ist. Die Lasche 18 kann eine erhöhte Flexibilität bei dem zu bildenden Umfang des Kühlpads an eine Extremität, um welche das Kühlpad vorgesehen werden soll, ermöglichen. In etwa horizontal gegenüberliegend an dem Kühlpad sind jeweils 2 fixierte Elemente 15, 15' oder 16 vorgesehen, wobei die in einer Ebene liegenden Elemente jeweils ein Verschlusselement bilden. Dies kann beispielsweise eine Kombination eines Klettverschlusses aus Haken und einem Material sein, in welchem sich die Haken verhaken können, wie dies beispielsweise bei Klettverschluss-Turnschuhen bekannt ist. Grundsätzlich kann das Hakenelement des Klettverschlusses sowohl auf der rechten als auch auf der linken Seite des Kühlpads vorgesehen sein.

## Patentansprüche

1. Kühlpad zur Kältebehandlung von Körperoberflächen mit
einem Beutel, welcher mit mindestens einer Kammer gebildet ist,
**dadurch gekennzeichnet,**
**dass** die mindestens eine Kammer mit einem Superabsorber für Wasser gefüllt ist und
**dass** der Beutel von außen nach innen wasserdurchlässig ist.

2. Kühlpad nach Anspruch 1,
**dadurch gekennzeichnet,**
**dass** der Superabsorber vernetzte Polyacrylate, insbesondere Polyacrylamid-Copolymere, und/oder Stärke-Copolymere und/oder Carboxymethylcellulose und/oder Hydroxymethylcellulose und/oder modifizierter Cellulose aufweist, insbesondere aus diesen ausgewählt ist.

3. Kühlpad nach einem der Ansprüche 1 oder 2,
**dadurch gekennzeichnet,**
**dass** die Kammern einen Anteil an Chlorid-Salz oder Polyvinylpyrrolidon aufweisen.

4. Kühlpad nach einem der Ansprüche 1 oder 3,
**dadurch gekennzeichnet,**
**dass** der Beutel zumindest einen Anteil an Polyester, vorzugsweise einen Anteil an Polyestergewebe, aufweist, besonders bevorzugt aus Polyestergewebe gebildet ist.

5. Kühlpad nach einem der Ansprüche 1 bis 4,
**dadurch gekennzeichnet,**
**dass** der Beutel zumindest einen Anteil an Polytetrafluorethylen, vorzugsweise einen Anteil an Gewebe mit Polytetrafluorethylenfasern, aufweist, besonders bevorzugt aus einem Polytetrafluorethylengewebe gebildet ist.

6. Kühlpad nach einem der Ansprüche 1 bis 5,
**dadurch gekennzeichnet,**
**dass** der Superabsorber als gepresstes Band, als eine Gitterstruktur, in Form eines Granulats oder Gels vorliegt.

7. Kühlpad nach einem der Ansprüche 1 bis 6,
**dadurch gekennzeichnet,**
**dass** der Beutel von innen nach außen für Wasserdampf jedoch nicht für Wasser durchlässig ist.

8. Verwendung eines Kühlpads nach einem der Ansprüche 1 bis 7 zur Behandlung von Schwellungen, Entzündungen und kühlbedürftigen Körperpartien.
